# EUROPÄISCHE PATENTSCHRIFT

(11) **EP 1 871 273 B1**
(45) Veröffentlichungstag und Bekanntmachung des Hinweises auf die Patenterteilung: **03.10.2018**
(21) Anmeldenummer: 06707498.9
(22) Anmeldetag: 09.03.2006
(51) Int. Cl.: A61C 1/18, A61C 1/12, A61B 17/16

(54) **MEDIZINISCHES ODER DENTALMEDIZINISCHES HANDSTÜCK**
MEDICAL OR DENTAL HANDPIECE
PIÈCE À MAIN MÉDICALE OU DENTAIRE

(30) Priorität: 09.03.2005 DE 102005010881
(43) Veröffentlichungstag der Anmeldung: 02.01.2008
(73) Patentinhaber: Kaltenbach & Voigt GmbH, 88400 Biberach (DE)
(72) Erfinder: KUHN, Bernhard, 88400 Biberach (DE)
(74) Vertreter: Thun, Clemens
(86) Internationale Anmeldenummer: PCT/EP2006/002186
(87) Internationale Veröffentlichungsnummer: WO 2006/094812

(56) Entgegenhaltungen:
- EP-A1- 0 405 132
- WO-A1-95/08960
- CH-A- 561 870
- GB-A- 760 983
- US-A- 4 355 977

## Beschreibung

Die vorliegende Erfindung betrifft ein medizinisches oder dentalmedizinisches Handstück mit einem Werkzeug, wobei ein Antriebsmechanismus für das Werkzeug zumindest ein mittels einer Lageranordnung drehbar gelagertes Drehteil aufweist und die Lageranordnung zumindest ein Kugellager mit mehreren zwischen einer Innenlaufbahn und einer Außenlaufbahn angeordneten Kugeln umfasst. Ferner betrifft die vorliegende Erfindung eine Lageranordnung, welche insbesondere zur Verwendung bei einem medizinischen oder dentalmedizinischen Handstück vorgesehen ist, um ein Drehteil drehbar zu lagern.

Medizinische oder dentalmedizinische Handstücke gibt es in unterschiedlichsten Ausgestaltungen hinsichtlich der Formgebung und Konstruktion, der Werkzeugart und -bewegung sowie der Antriebsart. Es sind bspw. Handstücke in Form eines sich gerade oder abgewinkelt erstreckten Griffteils bekannt. Bei dem zur Behandlung vorgesehenen Werkzeug kann es sich bspw. um ein Rotationswerkzeug oder um ein hin- und herbewegbares Werkzeug handeln. Als Antrieb hierfür kann im Handstück ein mechanischer Antrieb mit einer drehbar gelagerten Antriebswelle oder ein pneumatischer Antrieb mit einer vorzugsweise im vorderen Handstückbereich angeordneten Turbine ausgebildet sein, zu der es sich von hinten nach vorne eine Druckluftleitung erstreckt.

Medizinische, insbesondere zahnmedizinische Handstücke können somit in jedem Bereich ihrer Länge ein drehbar gelagertes Drehteil aufweisen, das für hochtourigen bis niedrigtourigen Funktionsbetrieb ausgebildet sein kann. Ein hochtouriger Funktionsbetrieb wird in den meisten Fällen für ein Werkzeug zur spanabhebenden Bearbeitung, z.B. zur Entfernung von Karies verwendet. Es gibt jedoch auch Handstücke mit einem Werkzeug, das mit niedrigerer Drehzahl angetrieben wird. Beispielsweise bei Werkzeugen, die im Funktionsbetrieb eine Schraubarbeit ausführen, wie es bei der Implantologie zum Setzen und Entfernen von dentalen Implantaten der Fall ist, liegen deutlich niedrigere Drehzahlen aber dafür ggf. höhere Drehmomente während des Betriebs vor.

Die drehbare Lagerung des Drehteils erfolgt mit Hilfe von Lagern, welche eine Rotation des Drehteils im Hinblick auf ein Basisteil des Handstücks ermöglichen.

Neben einem klassischen Kugellager ist auch die Verwendung sog. Nadellager oder anderweitig ausgestalteter Lager bekannt. In einigen Fällen ist sogar die Verwendung eines sog. Luftlagers denkbar. In den überwiegenden Anwendungsfällen kommen allerdings sog. Kugellager zum Einsatz, welche mehrere Kugeln aufweisen, die zwischen einem Innenring und einem Außenring angeordnet sind. Innen- und Außenring bilden dabei jeweils eine sog. Laufbahn, wobei sich die Kugeln innerhalb der beiden Laufbahnen fortbewegen können. Oftmals kommen auch noch sog. Käfige zum Einsatz, welche die Kugeln in einem vorbestimmten Abstand zueinander halten, so dass sich die Kugeln bei einer Rotation des Innenrings gegenüber dem Außenring in einem gleichbleibenden Abstand entlang der Laufbahnen bewegen. Sowohl für Innen-und Außenring als auch für die Kugeln können verschiedene Materialien verwendet werden. Üblicherweise bestehen diese aus Stahl, allerdings ist auch der alternative Einsatz von Keramikmaterialien insbesondere für die Kugeln bekannt.

Bei medizinischen oder dentalmedizinischen Handstücken kommen derartige Kugellager üblicherweise lediglich in miniaturisierter Form zum Einsatz, da die beengten Platzverhältnisse innerhalb der Handstücke lediglich die Nutzung von Lagern geringer Baugröße ermöglichen. Dies bedeutet allerdings wiederum, dass die Belastungen für die Lager nicht zu hoch sein dürfen, da die schmalen Innen- und Außenringe sowie die kleineren Kugeln nur Belastungen bis zu einem gewissen Grad aushalten.

Es hat sich nunmehr herausgestellt, dass die Belastungen für derartige Lager bei Handstücken, die für einen chirurgischen Einsatz vorgesehen sind, sehr hoch sind. Insbesondere in solchen Fällen, in denen zwar niedrige Drehzahlen aber hohe Drehmomente vorliegen, sind die Belastungen sogar derart groß, dass klassische Kugellager nicht mehr verwendet werden können. Üblicherweise werden bei derartigen Handstücken deshalb Gleitlager verwendet, die allerdings zu hohen Reibungsverlusten führen.

Aus dem Stand der Technik sind Lösungen zur Realisierung von Lagern für zahnärztliche Handstücke bekannt, bei denen die einander zugewandten Stirnflächen zweier Lagerteile als Laufflächen für die Kugeln genutzt werden. In der WO 95/08960 A1 erfolgt hierdurch die Lagerung eines Turbinenläufers im Kopfteil eines Turbinenhandstücks, in der EP 0 405 132 A1 wird mit Hilfe derartiger Lager eine von einem Motor angetriebene Welle in der Griffhülse eines Handstücks gelagert. Eine Lageranordnung in einem geraden Handstück ist aus der US 4 355 977 A bekannt.

Der vorliegenden Erfindung liegt die Aufgabe zugrunde, eine Möglichkeit anzugeben, Drehteile bei medizinischen oder dentalmedizinischen Handstücken in einfacher und effektiver Weise zu lagern. Insbesondere soll eine Lageranordnung geschaffen werden, welche geringe Reibungsverluste aufweist aber möglichst gut belastbar ist.

Die Aufgabe wird durch ein medizinisches oder dentalmedizinisches Handstück mit den Merkmalen des Anspruchs 1 gelöst. Vorteilhafte Weiterbildungen der Erfindung sind Gegenstand der Unteransprüche.

Gemäß der vorliegenden Erfindung wird somit ein medizinisches oder dentalmedizinisches Handstück mit einer Griffhülse vorgeschlagen, die abgewinkelt ausgeführt ist mit einem Kopplungsstück sowie einem gegenüber dem Kopplungsstück abgewinkelten Zwischenstück, wobei am vorderen Ende der Griffhülse ein Kopfteil angeordnet ist,
wobei ein Antriebsmechanismus für ein in dem Kopfteil anordbares Werkzeug zumindest eine mittels einer Lageranordnung drehbar in der Griffhülse gelagerte Welle aufweist,
wobei die Lageranordnung mindestens ein Kugellager mit mehreren zwischen einer Innenlaufbahn und einer Außenlaufbahn angeordneten Kugeln umfasst,
wobei die Innenlaufbahn des Kugellagers durch die Stirnfläche eines sich mit der Welle drehenden ersten Lagerteils und die Außenlaufbahn durch die Stirnfläche eines gegenüber der Welle fixierten und sich nicht mit dieser drehenden zweiten Lagerteils gebildet ist, wobei das zweite Lagerteil durch ein sich in der Griffhülse erstreckendes Zwischenstück gebildet ist, welches mit dem Kopfteil gekoppelt ist,
wobei die die Innenlaufbahn und die Außenlaufbahn bildenden Stirnflächen der beiden Lagerteile im Querschnitt in etwa viertelkreisförmig geformt sind,
wobei mindestens eines der beiden Lagerteile in Richtung des anderen Lagerteils axial vorgespannt ist,
und wobei das die Innenlaufbahn bildende Lagerteil gleichzeitig auch ein Kopplungsstück zur Kopplung mit einem Antriebsmechanismus innerhalb des Kopfteils des Handstücks bildet.

Die erfindungsgemäße Lösung gestattet es, die Laufbahnen für das Lager in einfacher Weise durch abhebendes Bearbeiten der Stirnflächen der Lagerteile, insbesondere durch Drehen zu gestalten. Dies ist nicht nur im Hinblick auf die bei der Herstellung der Teile anfallenden Kosten von Vorteil, es hat sich darüber hinaus auch gezeigt, dass hervorragende Laufeigenschaften erzielt werden und die Lager insbesondere eine hohe Belastbarkeit, auch bei kleinster Bauform aufweisen. Die Laufeigenschaften der Lager sind dabei derart vorteilhaft, dass sogar auf die Nutzung eines separaten Käfigs zur definierten Anordnung der Kugeln innerhalb der Laufbahnen verzichtet werden kann. Es wird somit eine besonders einfache Bauform für das Lager erzielt, welche im Hinblick auf die Herstellungskosten sowie die Betriebseigenschaften deutliche Vorteile gegenüber klassischen Kugellagern aufweist.

Die Vorspannung kann bspw. mittels einem Federelement erfolgen, welches zwischen dem vorzuspannenden Lagerteil und einer Schulter- oder Stirnfläche eines die Welle umgreifenden Zwischenstücks angeordnet ist. Dieses Federelement kann insbesondere durch eine Federscheibe gebildet sein.

Die Welle kann an ihren beiden Enden jeweils über ein Kugellager gelagert sein, welches entsprechend der vorliegenden Erfindung ausgestaltet ist. Darüber hinaus könnte allerdings noch eine zusätzliche Lagerung über ein sog. Nadellager erfolgen. In diesem Fall sind die Wälzkörper des Nadellagers zwischen der Außenwand des Drehteils und der Innenwand einer ringförmigen Ausnehmung des Zwischenstücks angeordnet.

Nachfolgend soll die Erfindung anhand der beiliegenden Zeichnungen näher erläutert werden. Es zeigen:
- Fig. 1: ein dentalmedizinisches Handstück im seitlichen Schnitt, bei dem eine erfindungsgemäße Lageranordnung zum Einsatz kommt;
- Fig. 2: eine vergrößerte Darstellung des Kopfbereichs des Handstücks von Fig. 1;
- Fig. 2a: eine Detaildarstellung des vorderen Lagers in Fig. 2;
- Fig. 3: ein Detail der erfindungsgemäßen Lageranordnung;
- Fig. 4: die drehbare Lagerung einer Welle mit Hilfe einer erfindungsgemäßen Lageranordnung;
- Fig. 5: eine Variante der Anordnung von Fig. 4 und
- Fig. 6: eine weitere Variante zur erfindungsgemäßen Lagerung einer Welle.

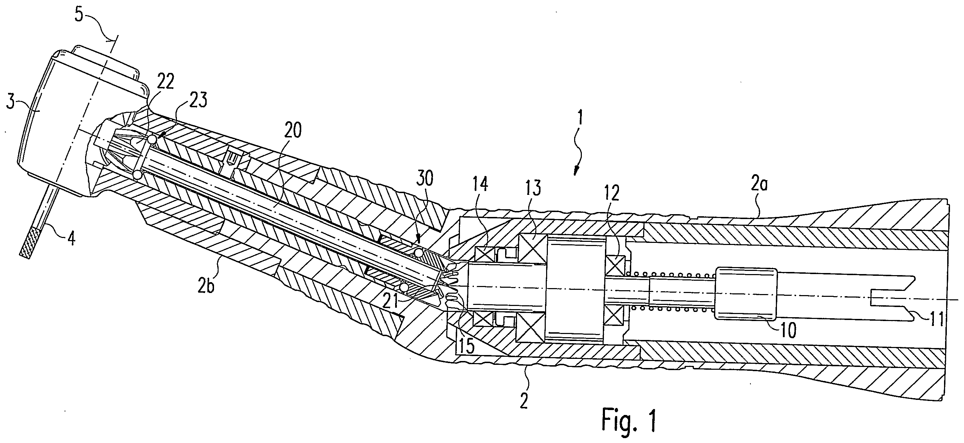

Fig. 1 zeigt ein allgemein mit dem Bezugszeichen 1 bezeichnetes zahnärztliches Handstück, welches eine abgewinkelte Griffhülse 2 mit einem Kopplungsstück 2a und einem Zwischenstück 2b aufweist, an deren vorderen Ende ein Handstückkopf 3 angeordnet ist. Innerhalb des Handstückkopfs 3 ist ein zahnärztliches Behandlungswerkzeug 4 um eine Achse 5 drehbar gelagert. Im dargestellten Ausführungsbeispiel handelt es sich insbesondere um ein zahnärztliches Chirurgiehandstück, bei dem das Werkzeug 4 mit niedrigen Drehzahlen aber bei verhältnismäßig hohen Drehmomenten betrieben wird.

Der Antrieb des Werkzeugs 4 erfolgt mit Hilfe eines Motors, der innerhalb eines nicht dargestellten Motorstücks angeordnet ist, mit dem das in Fig. 1 dargestellte Handstück 1 koppelbar ist. Mittels nicht näher dargestellter Kupplungselemente wird hierzu das Handstück 1 auf das Motorstück aufgesetzt, wobei dann ein Mitnehmer 11 eines Antriebsmechanismusses des Handstücks 1 mit dem Motor gekoppelt wird und dementsprechend über den Antriebsmechanismus das Werkzeug 4 in Rotation versetzt wird. Die einzelnen Komponenten des Antriebsmechanismus sollen nachfolgend erläutert werden.

Zunächst besteht der Antriebsmechanismus aus einer ersten Welle 10, die in dem Kopplungsstück 2a der Hülse 2 angeordnet ist und an ihrem rückwärtigen Ende den Mitnehmer 11 zur Kopplung mit dem Motor aufweist. Die Lagerung dieser Welle 10 erfolgt innerhalb des Kopplungsstücks 2a mittels mehrerer Lager 12, 13 und 14, bei denen es sich bspw. um Kugellager handeln kann. In diesem Kopplungsstück 2a ist der Einsatz von Kugellagern noch möglich, da hier die Platzverhältnisse den Einsatz von Lagern größerer Bauweise zulassen.

Eine zweite Welle 20 ist innerhalb des gegenüber dem Kopplungsstück 2a abgewinkelten Zwischenstücks 2b angeordnet. Die Aufgabe dieser zweiten Welle 20 ist es, die Rotation der ersten Welle 10 in das Kopfstück 3 zu übertragen, um dort eine Rotation des Werkzeug 4 hervorzurufen. Hierzu weist die zweite Welle 20 an ihrem rückwärtigen Ende erste Kopplungsteile 21 auf, die mit Kopplungsteilen 15 der Welle 10 zusammenwirken. An ihrem vorderen Ende weist die zweite Welle 20 ferner Kopplungsteile 22 auf, welche mit nicht näher dargestellten Kopplungsteilen innerhalb des Kopfstücks 3 zusammenwirken.

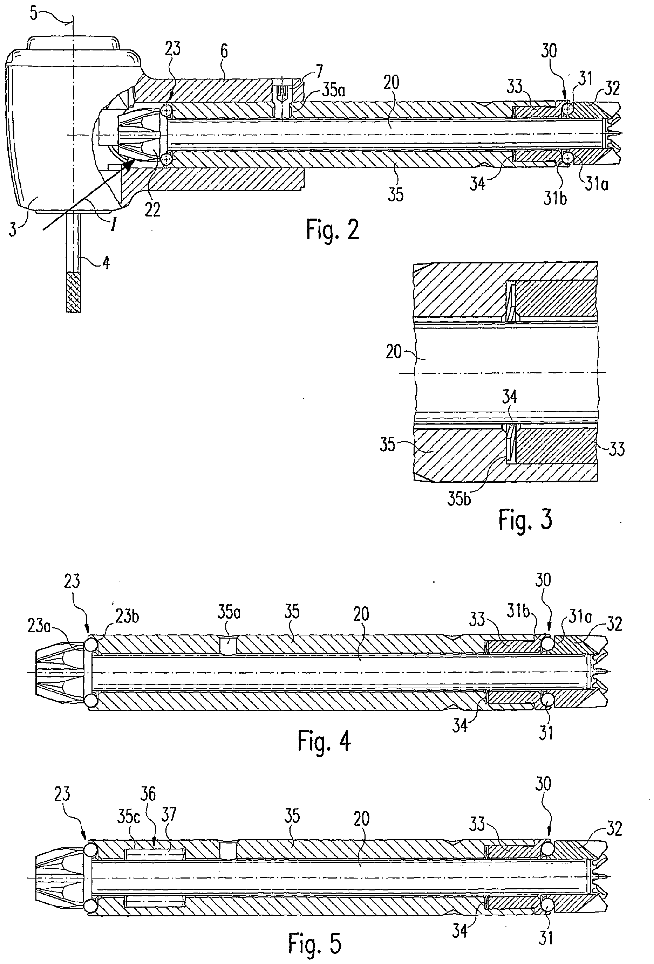

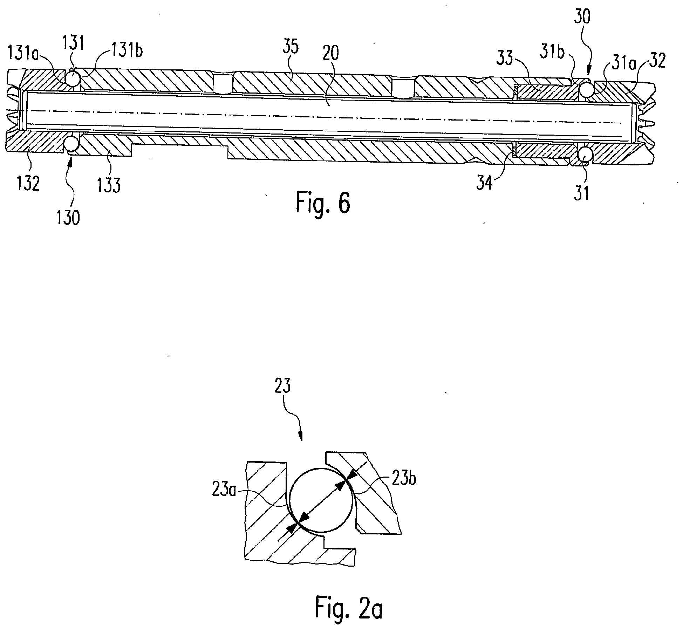

Die Lagerung der zweiten Welle 20 erfolgt mittels zweier Lager 23 und 30, deren nähere Ausgestaltung Fig. 2 entnommen werden kann. Zu berücksichtigen hierbei ist, dass die Platzverhältnisse innerhalb des Zwischenstücks 2b nur noch die Verwendung von Lagern geringerer Baugröße zulassen. Da allerdings insbesondere im rückwärtigen Bereich der Welle 20 höhere Belastungen auftreten, wird erfindungsgemäß zur Lagerung der Welle 20 in diesem Bereich eine besondere Lageranordnung 30 verwendet, die nachfolgend anhand der Fig. 2 und 3 erläutert werden soll.

Die erfindungsgemäße Lageranordnung 30 ist wiederum als Kugellager ausgestaltet und weist dementsprechend mehrere, vorzugsweise 14 Kugeln 31 auf, welche zwischen zwei mit den Bezugszeichen 31a und 31b versehenen Laufbahnen geführt sind und eine Rotation der Welle 20 gegenüber einem fixierten sog. Zwischenstück 35, welches sich innerhalb der Griffhülse 2 erstreckt, ermöglichen. Das Zwischenstück 35 ist mit dem Kopfteil 3 des Handstücks gekoppelt, wozu das Kopfteil eine zur Rückseite abstehende Hülse 6 aufweist, welche das Zwischenstück 35 übergreift. Im hinteren Endbereich weist die Hülse 6 einen Raststift 7 auf, der in eine Ausnehmung 35a des Zwischenstücks eingreift und hierdurch eine Verrastung und axiale Befestigung der beiden Teile bewirkt.

Zur Bildung eines klassischen Kugellagers werden die Innen- und Außenlaufbahn für die Kugeln üblicherweise durch zwei Lagerringe gebildet, welche an ihrer äußeren Mantelfläche bzw. an ihrer Innenfläche ringförmige Vertiefungen oder Ausnehmungen aufweisen, welche die Laufbahnen bilden. Auch bei der erfindungsgemäßen Lageranordnung werden die Laufbahnen 31a und 31b durch entsprechend ausgestaltete Lagerteile 32 und 33 gebildet, wobei die Laufflächen nunmehr allerdings nicht an der äußeren Mantelfläche bzw. an der Innenfläche der beiden Lagerteile sondern stattdessen an deren Stirnflächen gebildet sind.

Wie der Darstellung in Fig. 2 entnommen werden kann, sind beide Stirnflächen jeweils im Querschnitt gesehen viertelkreisförmig gestaltet, wodurch einander zugewandte Laufflächen gebildet werden, welche einerseits an den der Welle 20 zugewandten Außenflächen sowie andererseits an den gegenüberliegenden Außenflächen der Kugeln 31 anliegen. Die Stirnfläche des ersten Lagerteils 32, welches fest an der Welle 20 angeordnet ist und sich mit dieser dreht, bildet dementsprechend eine Innenlaufbahn 31a für die Kugeln 31, während hingegen die Stirnfläche des zweiten Lagerteils 33 eine Außenlaufbahn 31b für die Kugeln 31 bildet. Das zweite Lagerteil 33 wird ebenfalls durch ein hülsenförmiges Teil gebildet, welches sich allerdings nicht mit der Welle 20 dreht sondern stattdessen gegenüber dieser fest angeordnet ist.

Die Stirnflächen beider Lagerteile 32 und 33 bilden somit Laufbahnen, welche gemeinsam die Kugeln 31 nahezu vollständig umgreifen. Hierdurch wird eine sehr stabile Anordnung für das Lager erzielt, welche es sogar gestattet, auf den üblicherweise verwendeten Käfig zur definierten Anordnung der Kugeln 31 innerhalb der Laufbahnen zu verzichten. Die Gestaltung der Stirnflächen der beiden Lagerteile 32 und 33 kann bspw. durch entsprechendes Drehen erfolgen, was auch Vorteile hinsichtlich des Kostenaufwands bei der Erstellung des Lagers mit sich bringt.

Gute Laufeigenschaften für die erfindungsgemäße - käfiglose - Lageranordnung 30 werden insbesondere dann erzielt, wenn sichergestellt ist, dass die Kugeln 31 fest innerhalb ihrer Laufbahnen 31a und 31b angeordnet sind. Es ist dementsprechend vorgesehen, dass eines der beiden Lagerteile 32, 33 gegenüber dem anderen in Axialrichtung vorgespannt ist. Im dargestellten Ausführungsbeispiel erfolgt dies dadurch, dass an der rückwärtigen. Stirnfläche des zweiten Lagerteils 33 eine Federscheibe 34 angeordnet ist, welche - wie insbesondere der vergrößerten Darstellung von Fig. 3 entnommen werden kann - gegen eine Stirnfläche bzw. eine Schulter 35b des Zwischenstücks 35 drückt. Auf diese Weise wird das zweite Lagerteil 33 in Richtung des ersten Lagerteils 32 vorgespannt, so dass eine sichere Halterung der Kugeln 31 innerhalb ihrer Laufbahnen 31a und 31b erzielt wird. Die sich hierbei insgesamt ergebende Lagerung der Welle 20 kann nochmals der Darstellung in Fig. 4 entnommen werden.

Im übrigen ist auch das dem Handstückkopf 3 zugewandte Lager 23 in erfinderischer Weise ausgestaltet. Auch bei diesem Lager 23 bilden somit die einander zugewandten Stirnflächen des mit der Welle 20 verbundenen Kopplungsteils 22 und des Zwischenstücks 35 Laufbahnen 23a, 23b für die Kugeln. In diesem Zusammenhang ist auch darauf hinzuweisen, das die erfindungsgemäße Ausgestaltung des Lagers besondere Vorteile hinsichtlich der Krautaufnahme mit sich bringt. Wie nämlich in Fig. 2 schematisch durch den Pfeil I dargestellt ist, ergibt sich im Betrieb des Handstücks eine Hauptbelastungsrichtung, die aus Richtung des Bohrers 4 schräg nach oben zum Lager 23 hin verläuft. Entsprechend der vergrößerten Darstellung von Fig. 2a ist nunmehr die Ausgestaltung der Laufbahnen derart, dass die Kugeln genau in Kraftrichtung umschlossen werden. Es ergibt sich also eine ideale Kraftaufnahme durch das Lager, die zu einer weiteren Verbesserung der Laufeigenschaften führt.

Besondere Vorteile der erfindungsgemäßen Lageranordnung 30 bestehen ferner darin, dass einerseits wenig Bauteile zum Einsatz kommen und andererseits die die Laufbahnen 31a und 31b bildenden Lagerteile 32 und 33 in einer gewissen Größe bzw. Breite ausgestaltet sein können. Hierdurch besteht also die Möglichkeit, die Lagerteile zumindest derart auszugestalten, dass sie auch größeren Belastungen, die z.B. bei einem Betrieb mit hohen Drehmomenten auftreten könnten, standhalten. Im Vergleich zu herkömmlichen Kugellagern mit miniaturisierten Innen- und Außenringen kann die erfindungsgemäße Lageranordnung also deutlich größere Kräfte aufnehmen. Trotz allem sind die Laufeigenschaften der erfindungsgemäßen Anordnung zumindest vergleichbar mit klassischen Kugellagern, so dass auch der Einsatz bei Drehzahlen im Bereich von bis zu 40.000 Umdrehungen pro Minute denkbar wäre. Entscheidend für die guten Eigenschaften der erfindungsgemäßen Anordnung ist insbesondere auch die integrierte Anstellung beider Lagerteile zueinander, welche durch die Vorspannung mit Hilfe der Federscheibe erfolgt. Selbstverständlich sollten sämtliche Teile der Lageranordnung aus einem gehärteten Material, beispielsweise Stahl bestehen, wobei allerdings beispielsweise auch der Einsatz von Keramikkugeln denkbar wäre.

Fig. 5 zeigt eine Variante zur Lagerung der Welle 20 gemäß der Darstellung in Fig. 4. Bei der Variante gemäß Fig. 5 ist zusätzlich ein Nadellager 36 im vorderen Endbereich der Welle 20 vorgesehen, welches zur zusätzlichen Lagerung der Welle 20 eingesetzt wird. Die Wälzkörper 37 des Nadellagers 36 sind in diesem Fall zwischen der Außenwand der Welle 20 und der Innenwand einer ringförmigen Ausnehmung 35c des Zwischenstücks 35 angeordnet. Auch hier wird das rückwärtige Lager 30 durch die erfindungsgemäße Anordnung gebildet, bei der die Laufflächen durch die Stirnflächen der Lagerteile 32 und 33 gebildet werden.

Fig. 6 zeigt schließlich ein weiteres Ausführungsbeispiel zur Lagerung einer Welle 20, bei dem gleiche Elemente mit den gleichen Bezugszeichen versehen sind. Die Erweiterung des Ausführungsbeispiels gemäß Fig. 6 besteht darin, dass auch bei dem Lager 130 im vorderen Endbereich der Welle 20 ein zusätzliches Lagerteil zum Bilden der Innenlaufbahn vorgesehen ist. Wiederum sind somit zwei Lagerteile 132 und 133 zur Lagerung der Kugeln 131 vorgesehen, deren Stirnflächen die Innen- und Außenlaufbahnen 131a und 131b für die Kugeln 131 bilden. Das die Innenlaufbahn 131a bildende Lagerteil 132 stellt in diesem Fall gleichzeitig auch das Kopplungsstück zur Kopplung mit dem Antriebsmechanismus innerhalb des Kopfteils 3 des zahnärztlichen Handstücks 1 dar, während hingegen das zweite Lagerteil 133 durch das Zwischenstück 35 gebildet wird. Wiederum sind die beiden Stirnflächen der beiden Lagerteile 132 und 133 viertelkreisförmig ausgestaltet, so dass sie die Laufbahnen für die Kugeln 131 bilden.

Insgesamt wird somit gemäß der vorliegenden Erfindung eine neuartige Lageranordnung zur drehbaren Lagerung eines Drehteils insbesondere innerhalb eines medizinischen oder dentalmedizinischen Handstücks geschaffen, welche trotz geringer Baugröße hohen Belastungen ausgesetzt werden kann. Trotz allem werden hervorragende Laufeigenschaften erzielt, so dass das erfindungsgemäße Lager vielseitig einsetzbar ist und eine hohe Betriebssicherheit aufweist. Darüber hinaus gestattet die erfindungsgemäße Ausgestaltung der Lageranordnung auch eine kostengünstige Herstellung derselben.

## Patentansprüche

1. Medizinisches oder dentalmedizinisches Handstück (1) mit einer Griffhülse (2), die abgewinkelt ausgeführt ist, mit einem Kopplungsstück (2a) sowie einem gegenüber dem Kopplungsstück (2a) abgewinkelten Griffhülsen-Zwischenstück (2b), wobei am vorderen Ende der Griffhülse (2) ein Kopfteil angeordnet ist,
wobei ein Antriebsmechanismus für ein in dem Kopfteil anordbares Werkzeug (4) zumindest eine mittels einer Lageranordnung (30, 130) drehbar im Griffhülsen-Zwischenstück (2b) gelagerte Welle (20) aufweist,
wobei die Lageranordnung (30, 130) mindestens ein Kugellager mit mehreren zwischen einer Innenlaufbahn (31a, 131a) und einer Außenlaufbahn (31b, 131b) angeordneten Kugeln (31, 131) umfasst,
wobei die Innenlaufbahn (31a, 131a) des Kugellagers durch die Stirnfläche eines sich mit der Welle (20) drehenden ersten Lagerteils (32, 132) und die Außenlaufbahn (31b, 131b) durch die Stirnfläche eines gegenüber der Welle (20) fixierten und sich nicht mit dieser drehenden zweiten Lagerteils (33, 133) gebildet ist, wobei das zweite Lagerteil (33) durch ein sich in der Griffhülse (2) erstreckendes Lager-Zwischenstück (35) gebildet ist,
welches mit dem Kopfteil gekoppelt ist,
wobei die die Innenlaufbahn (31a, 131a) und die Außenlaufbahn (31b, 131b) bildenden Stirnflächen der beiden Lagerteile (32, 33; 132, 133) im Querschnitt in etwa viertelkreisförmig geformt sind,
wobei mindestens eines der beiden Lagerteile (32, 33; 132, 133) in Richtung des anderen Lagerteils (32, 33; 132, 133) axial vorgespannt ist,
und wobei das die Innenlaufbahn (31a, 131a) bildende Lagerteil (132) gleichzeitig auch ein Kopplungsstück zur Kopplung mit dem Antriebsmechanismus innerhalb des Kopfteils (3) des Handstücks (1) bildet.

2. Medizinisches oder dentalmedizinisches Handstück nach Anspruch 1,
**dadurch gekennzeichnet,**
**dass** die die Innenlaufbahn (31a, 131a) und die Außenlaufbahn (31b, 131b) bildenden Stirnflächen der beiden Lagerteile (32, 33; 132, 133) gemeinsam die Kugeln (31; 131) nahezu vollständig umgreifen.

3. Medizinisches oder dentalmedizinisches Handstück nach einem der vorherigen Ansprüche,
**dadurch gekennzeichnet,**
**dass** die Anordnung der Kugeln (31; 131) zwischen der Innenlaufbahn (31a, 131a) und der Außenlaufbahn (31b, 131b) käfiglos erfolgt.

4. Medizinisches oder dentalmedizinisches Handstück nach einem der vorherigen Ansprüche,
**dadurch gekennzeichnet,**
**dass** die Vorspannung mittels einem Federelement (34) erfolgt, welches zwischen dem entsprechenden Lagerteil (32, 33; 132, 133) und einer Schulter- oder Stirnfläche (35b) des die Welle (20) umgreifenden Lager-Zwischenstücks (35) angeordnet ist.

5. Medizinisches oder dentalmedizinisches Handstück nach Anspruch 4,
**dadurch gekennzeichnet,**
**dass** das Federelement durch eine Federscheibe (34) gebildet ist.

6. Medizinisches oder dentalmedizinisches Handstück nach einem der vorherigen Ansprüche,
**dadurch gekennzeichnet,**
**dass** das erste Lagerteil (32, 132) durch eine auf der Welle (20) angeordnete Lagerhülse gebildet ist.

7. Medizinisches oder dentalmedizinisches Handstück nach einem der vorherigen Ansprüche,
**dadurch gekennzeichnet,**
**dass** die beiden Lagerteile (32, 33; 132, 133) aus einem gehärteten Material, insbesondere aus gehärtetem Stahl bestehen.

8. Medizinisches oder dentalmedizinisches Handstück nach einem der vorherigen Ansprüche,
**dadurch gekennzeichnet,**
**dass** die Kugeln (31, 131) aus gehärtetem Stahl oder aus einem Keramikmaterial bestehen.

9. Medizinisches oder dentalmedizinisches Handstück nach einem der vorherigen Ansprüche,
**dadurch gekennzeichnet,**
**dass** die Welle (20) an beiden Enden über Kugellager gelagert ist.

10. Medizinisches oder dentalmedizinisches Handstück nach einem der vorherigen Ansprüche,
**dadurch gekennzeichnet,**
**dass** die Lagerung der Welle (20) zusätzlich über ein Nadellager (36) erfolgt.

11. Medizinisches oder dentalmedizinisches Handstück nach Anspruch 10,
**dadurch gekennzeichnet,**
**dass** die Wälzkörper (37) des Nadellagers (36) zwischen der Außenwand der Welle (20) und der Innenwand einer ringförmigen Ausnehmung (35c) des Lager-Zwischenstücks (35) angeordnet sind.

12. Medizinisches oder dentalmedizinisches Handstück nach einem der vorherigen Ansprüche,
**dadurch gekennzeichnet,**
**dass** es sich um ein um ein zahnärztliches Chirurgiehandstück handelt.

## Claims

1. A medical or dental-medical handpiece (1) having a grip sleeve (2) which is constructed in an angled manner, having a coupling piece (2a) and also a grip-sleeve intermediate piece (2b) that is angled with respect to the coupling piece (2a), wherein a head part is arranged at the forward end of the grip sleeve (2),
wherein a drive mechanism for a tool (4), which can be arranged in the head part, has at least one shaft (20) mounted rotatably by means of a bearing arrangement (30, 130) in the grip-sleeve intermediate piece (2b),
wherein the bearing arrangement (30, 130) includes at least one ball bearing with a plurality of balls (31, 131) arranged between an inner track (31a, 131a) and an outer track (31b, 131b),
wherein the inner track (31a, 131a) of the ball bearing is formed by means of the end face of a first bearing part (32, 132) rotating with the shaft (20), and the outer track (31b, 131b) is formed by means of the end face of a second bearing part (33, 133) fixed with respect to the shaft (20) and not rotating therewith,
wherein the second bearing part (33) is formed by a bearing intermediate piece (35) which extends within the grip sleeve (2) and is coupled to the head part,
wherein the end faces of the two bearing parts (32, 33; 132, 133) forming the inner track (31a, 131a) and the outer track (31b, 131b) are of approximately quarter circle shape, seen in cross-section,
wherein at least one of the two bearing parts (32, 33; 132, 133) is axially biased in the direction of the other bearing part (32, 33; 132, 133),
and wherein the bearing part (132) that forms the inner track (31a, 131a) at the same time also forms a coupling piece for the coupling with the drive mechanism within the head part (3) of the handpiece (1).

2. A medical or dental-medical handpiece according to claim 1,
**characterised in that**
the end faces of the two bearing parts (32, 33; 132, 133) forming the inner track (31a, 131a) and the outer track (31b, 131b) together almost completely engage around the balls (31; 131).

3. A medical or dental-medical handpiece according to one of the preceding claims,
**characterised in that**
the arrangement of the balls (31; 131) between the inner track (31a, 131a) and the outer track (31b, 131b) is effected without cages.

4. A medical or dental-medical handpiece according to one of the preceding claims,
**characterised in that**
the biasing is effected by means of a spring element (34) which is arranged between the corresponding bearing part (32, 33; 132, 133) and a shoulder or end face (35b) of the bearing intermediate piece (35) engaging around the shaft (20).

5. A medical or dental-medical handpiece according to claim 4,
**characterised in that**
the spring element is formed by a flat spring (34).

6. A medical or dental-medical handpiece according to one of the preceding claims,
**characterised in that**
the first bearing part (32, 132) is formed by a bearing sleeve arranged on the shaft (20).

7. A medical or dental-medical handpiece according to one of the preceding claims,
**characterised in that**
the two bearing parts (32, 33; 132, 133) consist of a hardened material, in particular of hardened steel.

8. A medical or dental-medical handpiece according to one of the preceding claims,
**characterised in that**
the balls (31, 131) consist of hardened steel or of a ceramic material.

9. A medical or dental-medical handpiece according to one of the preceding claims,
**characterised in that**
the shaft (20) is mounted at both ends via ball bearings.

10. A medical or dental-medical handpiece according to one of the preceding claims,
**characterised in that**
the mounting of the shaft (20) is additionally effected by way of a needle bearing (36).

11. A medical or dental-medical handpiece according to claim 10,
**characterised in that**
the roller bodies (37) of the needle bearing (36) are arranged between the outer wall of the shaft (20) and the inner wall of a ring-shaped recess (35c) of the bearing intermediate piece (35).

12. A medical or dental-medical handpiece according to one of the preceding claims,
**characterised in that**
it is a dental surgical handpiece.

## Revendications

1. Pièce à main (1) médicale ou médico-dentaire avec un manchon de préhension (2), qui est réalisé de manière coudée, avec une pièce de couplage (2a) ainsi qu'avec une pièce intermédiaire de manchon de préhension (2b) coudée par rapport à la pièce de couplage (2a), dans laquelle une partie de tête est disposée au niveau de l'extrémité avant du manchon de préhension (2),
dans laquelle un mécanisme d'entraînement pour un outil (4) pouvant être disposé dans la partie de tête présente au moins un arbre (20) monté de manière à pouvoir tourner dans la pièce intermédiaire de manchon de préhension (2b) au moyen d'un ensemble formant palier (30, 130),
dans laquelle l'ensemble formant palier (30, 130) comprend au moins un roulement à billes avec plusieurs billes (31, 131) disposées entre une voie de roulement intérieure (31a, 131a) et une voie de roulement extérieure (31b, 131b),
dans laquelle la voie de roulement intérieure (31a, 131a) du roulement à billes est formée par la face frontale d'une première partie de palier (32, 132) tournant avec l'arbre (20) et la voie de roulement extérieure (31b, 131b) est formée par la face frontale d'une deuxième partie de palier (33, 133) fixée en vis-à-vis de l'arbre (20) et ne tournant pas avec ce dernier, dans laquelle la deuxième partie de palier (33) est formée par une pièce intermédiaire de palier (35) s'étendant dans le manchon de préhension (2), qui est couplée à la partie de tête,
dans laquelle les faces frontales formant la voie de roulement intérieure (31a, 131a) et la voie de roulement extérieure (31b, 131b), des deux parties de palier (32, 33 ; 132, 133) sont formées dans la section transversale sous une forme à peu près de quart de cercle,
dans laquelle au moins une des deux parties de palier (32, 33 ; 132, 133) est précontrainte de manière axiale en direction de l'autre partie de palier (32, 33 ; 132, 133),
et dans laquelle la partie de palier (132) formant la voie de roulement intérieure (31a, 131a) forme de manière simultanée également une pièce de couplage destinée à être couplée au mécanisme d'entraînement à l'intérieur de la partie de tête (3) de la pièce à main (1).

2. Pièce à main médicale ou médico-dentaire selon la revendication 1,
**caractérisée en ce**
**que** les faces frontales, formant la voie de roulement intérieure (31a, 131a) et la voie de roulement extérieure (31b, 131b), des deux parties de palier (32, 33 ; 132, 133) entourent quasiment en totalité conjointement les billes (31 ; 131).

3. Pièce à main médicale ou médico-dentaire selon l'une quelconque des revendications précédentes,
**caractérisée en ce**
**que** l'agencement des billes (31 ; 131) est effectué sans cage entre la voie de roulement intérieure (31a, 131a) et la voie de roulement extérieure (31b, 131b).

4. Pièce à main médicale ou médico-dentaire selon l'une quelconque des revendications précédentes,
**caractérisée en ce**
**que** la précontrainte est effectuée au moyen d'un élément de ressort (34), qui est disposé entre la partie de palier (32, 33 ; 132, 133) correspondante et une face d'épaulement ou frontale (35b) de la pièce intermédiaire de palier (35) entourant l'arbre (20).

5. Pièce à main médicale ou médico-dentaire selon la revendication 4,
**caractérisée en ce**
**que** l'élément de ressort est formé par un disque-ressort (34).

6. Pièce à main médicale ou médico-dentaire selon l'une quelconque des revendications précédentes,
**caractérisée en ce**
**que** la première partie de palier (32, 132) est formée par un manchon de palier disposé sur l'arbre (20).

7. Pièce à main médicale ou médico-dentaire selon l'une quelconque des revendications précédentes,
**caractérisée en ce**
**que** les deux parties de palier (32, 33 ; 132, 133) sont constituées d'un matériau durci, en particulier d'un acier durci.

8. Pièce à main médicale ou médico-dentaire selon l'une quelconque des revendications précédentes,
**caractérisée en ce**
**que** les billes (31, 131) sont constituées d'un acier durci ou d'un matériau en céramique.

9. Pièce à main médicale ou médico-dentaire selon l'une quelconque des revendications précédentes,
**caractérisée en ce**
**que** l'arbre (20) est monté au niveau des deux extrémités par l'intermédiaire de roulements à billes.

10. Pièce à main médicale ou médico-dentaire selon l'une quelconque des revendications précédentes,
**caractérisée en ce**
**que** le montage de l'arbre (20) est effectué en supplément par l'intermédiaire d'un roulement à aiguilles (36).

11. Pièce à main médicale ou médico-dentaire selon la revendication 10,
**caractérisée**
**en ce que** les corps de roulement (37) du roulement à aiguilles (36) sont disposés entre la paroi extérieure de l'arbre (20) et la paroi intérieure d'un évidement (35c) de forme annulaire de la pièce intermédiaire de palier (35).

12. Pièce à main médicale ou médico-dentaire selon l'une quelconque des revendications précédentes,
**caractérisée en ce**
**qu'**il s'agit d'une pièce à main chirurgicale dentaire.
